Europäisches Patentamt

European Patent Office

Office européen des brevets

(11) Veröffentlichungsnummer: **0 135 099**
**A2**

(12) # EUROPÄISCHE PATENTANMELDUNG

(21) Anmeldenummer: **84109278.6**

(22) Anmeldetag: **04.08.84**

(51) Int. Cl.⁴: **C 12 P 19/44**

(30) Priorität: **09.08.83 CH 4327/83**

(43) Veröffentlichungstag der Anmeldung:
**27.03.85 Patentblatt 85/13**

(84) Benannte Vertragsstaaten:
**AT BE CH DE FR GB IT LI LU NL SE**

(71) Anmelder: **Technolizenz Establishment**
**Austrasse 4**
**FL-9490 Vaduz(LI)**

(72) Erfinder: **Käppeli, Othmar**
**Bachwiesenstrasse 40**
**CH-8116 Würenlos(CH)**

(72) Erfinder: **Guerra-Santos, Luis**
**Dörflistrasse 75**
**CH-8050 Zürich(CH)**

(74) Vertreter: **Büchel, Kurt F., Dr.**
**Austrasse 4**
**FL-9490 Vaduz(LI)**

(54) **Verfahren zur Herstellung von Tensiden.**

(57) Zur Herstellung von als Tenside verwendbaren Rhamnolipiden werden Mikroorganismen der Gattung Pseudomonas
in einem für das Wachstum der Mikroorganismen geeigneten wässrigen Kulturmedium unter Wachstumsbedingungen
der Mikroorganismen kultiviert; dabei werden die Mikroorganismen in einer kontinuierlichen Submerskultur unter
aeroben Bedingungen und mit fortlaufender Zuführung von
frischem Kulturmedium sowie fortlaufender Abführung einer
Kulturmasse aus Kulturmedium und mikrobiellen Zellen
gezüchtet; das Kulturmedium hat eine zur Wachstumsbegrenzung durch mindestens zweifache Limitierung essentieller Wachstumsstoffe geeignete Zusammensetzung; von der
Kulturmasse wird eine Lösung der als Metabolit der Mikroorganismen gebildeten Rhamnolipide abgetrennt. Hierzu ist
ein kontinuierlich betriebener Bioreaktor (11) geeignet.

./...

FIGUR 1

## Verfahren zur Herstellung von Rhamnolipiden

Die Erfindung betrifft ein mikrobiologisches Verfahren zur
Herstellung von als Tenside verwendbaren Rhamnolipiden.

Es ist bekannt, dass mikrobielles Wachstum auf einem Substrat
durch Tenside, d. h. natürliche oder synthetische oberflächenaktive bzw. grenzflächenaktive Stoffe, begünstigt wird, wenn
die Tenside die Zugänglichkeit des Substrates für die Mikroorganismen erhöhen. Hierfür geeignete Tenside können von den
Mikroorganismen selbst gebildet oder als Hilfsmittel, z. B. in
Form von synthetischem Tensid, zugegeben werden; die metabolitisch von den Mikroorganismen selbst erzeugten Tenside werden
neuerdings als Biotenside bezeichnet und können unterschiedlichen Stoffklassen angehören.

Seit man insbesondere durch Untersuchung von Rückständen aus
Mineralölschlamm Mikroorganismen kennt, die Mineralöl bzw.
Kohlenwasserstoffe als Substrat für das Wachstum ausnützen
können, ist man bemüht, mikrobielle Methoden für die Verbesserung der Erdölgewinnung einzusetzen und hat in diesem Zusammenhang den für den mikrobiellen Abbau von Kohlenwasserstoffen offensichtlich bedeutsamen Biotensiden erhöhte Beachtung geschenkt.

So ist es beispielsweise aus US-PS 3'340'930 und 4'096'073
bekannt, mineralölhaltige Erdschichtbereiche mit einem tensidhaltigen wässrigen Medium zu fluten, das vorgängig durch
Züchten bestimmter, zum Wachstum auf Kohlenwasserstoffen befähigter Mikroben aus der Klasse der Schizomyceten oder Hefen aus der Ordnung der Saccharomycetalen auf einem kohlenwasserstoffhaltigen Kulturmedium gebildet worden ist. Gemäss
US-PS 4'286'660 sind zum Fluten von Erdöllagern besonders
die als Glycolipide bezeichneten Biotenside geeignet, die in
wässrigem Medium mit Kohlenwasserstoff als C-Quelle gebildet
werden; dabei kann in bekannten Bioreaktoren gearbeitet werden, die einen halbkontinuierlichen oder vollkontinuierlichen Flutungsbetrieb gestatten. Im kontinuierlichen Betrieb
wird beispielsweise so verfahren, dass das Biotensid enthaltende Flutwasser durch ein erstes Bohrloch in das Mineralöllager hineingepumpt und über ein zweites Bohrloch zusammen
mit ausgespültem Mineralöl abgezogen wird. Das abgezogene
Flutwasser kann nach Abtrennung des überwiegenden Mineralölanteils wieder in den Bioreaktor zurückgeführt und zur Bildung von Glycolipid verwendet werden.

Obwohl für die Erdölgewinnung nach solchen Methoden der verstärkten Oelgewinnung ("enhanced oil recovery") auch die üblichen synthetischen Tenside verwendbar sind und verwendet werden, bieten Biotenside, wie z. B. die Glycolipide, für diese und andere Anwendungen aufgrund ihrer angepassten bzw. natürlichen Entstehung besondere Vorteile.

Allgemein besteht ein Bedarf an neuen Tensiden und/oder verbesserten Tensidherstellungsverfahren. Zwar kennt man zahlreiche synthetische Tenside, doch sind diese z. B. bezüglich des Verhältnisses von Wirksamkeit zu biologischer Abbaubarkeit und/oder bezüglich Temperaturstabilität sowie Scherkraftempfindlichkeit oder physiologischer Wirkungen durchaus verbesserungsfähig. Da es kein synthetisches Tensid gibt, das für alle Zwecke geeignet wäre, sind auch aus Gründen der Spezialisierung zusätzliche Tenside nötig.

Man sucht daher weiterhin nach neuen Tensiden und es wäre wünschbar, die bisher hauptsächlich für die Erdölförderungstechnik vorgeschlagenen Methoden der Biotensidgewinnung auch zur Herstellung von Tensiden für andere Verwendungsformen einzusetzen. Die zur vorliegenden Erfindung führenden Untersuchungen haben jedoch gezeigt, dass eine wirtschaftlich durchführbare Gewinnung von als oberflächenaktive Substanzen (z. B. zur Verwendung als Suspensions-, Dispergier- und Netzmittel oder dergleichen für kommerzielle Verfahren bzw. Produkte) geeigneten Biotensiden mit den bekannten Kulturmedien, -methoden und Mikroorganismen wahrscheinlich nicht oder nur schwer erreichbar ist.

- 3a -

0135099

Es wurde gefunden, dass Rhamnolipide der von Itoh, S. et al, J. Antibiot. 24: 855-859, beschriebenen und durch Züchtung von Mikroorganismen der Gattung Pseudomonas, insbesondere Pseudomonas aeruginosa, auf n-Paraffinen erhältlichen Art als Biotenside vorteilhaft wären, aber gemäss Stand der Technik nur nach unwirtschaftlichen Methoden in Chargenbetrieb erhältlich sind, wie dies z. B. für verschiedene Pseudomonas-Stämme in der DE-OS 21 50 375 beschrieben ist.

Die zur Erfindung führenden Untersuchungen zeigten ferner, dass die kontinuierliche Züchtung aufgrund der Kenntnisse des Standes der Technik nicht zu brauchbaren Produkten bzw. Produktausbeuten führt.

Der Erfindung liegt nun die Aufgabe zugrunde, ein Verfahren anzugeben, nach welchem Rhamnolipide mit einer zur Verwendung als Tensid ausreichenden Grenzflächenaktivität in kontinuierlichem Verfahren und mit brauchbaren Ausbeuten erhalten werden können.

Diese Aufgabe wird erfindungsgemäss gelöst durch ein Verfahren zur Herstellung von als Tenside verwendbaren Rhamnolipiden durch Kultivieren von Mikroorganismen der Gattung Pseudomonas, insbesondere Pseudomonas aeruginosa, in einem für das Wachstum dieser Mikroorganismen geeigneten wässrigen Kulturmedium unter Wachstumsbedingungen der Mikroorganismen, welches Verfahren dadurch gekennzeichnet ist, dass

(a) die Mikroorganismen in einer kontinuierlichen Submerskultur unter aeroben Bedingungen und mit fortlaufender Zuführung von frischem Kulturmedium sowie fortlaufender Abführung einer Kulturmasse aus teilweise verbrauchtem Kulturmedium und gebildeten Tensiden, sowie gegebenenfalls mikrobiellen Zellen;

(b) das Kulturmedium eine zur Wachstumsbegrenzung durch mindestens zweifache Limitierung essentieller Wachstumsstoffe geeignete Zusammensetzung aufweist.

Die Kulturmasse kann für bestimmte Anwendungsgebiete, z.B. für die Oelgewinnung, als solche verwendet werden, oder es wird von der Kulturmasse eine Lösung der als Metabolit der Mikroorganismen gebildeten Rhamnolipide abgetrennt.

Die in Schritt (c) erhaltene Lösung mit einem Gehalt an Rhamnolipid von typisch über 1 g pro Liter kann als solche oder in konzentrierter Form verwendet werden; gewünschtenfalls kann das Rhamnolipid nach an sich bekannten Verfahren, wie Fällen oder/und Affinitätschromatographie isoliert bzw. gereinigt werden, z. B. mit handelsüblichen Absorptionsmitteln aus synthetischen Polymeren, wie solchen auf Basis von Styrol oder Arylsäureestern.

Aus der oben genannten DE-OS 21 50 375 ist bekannt, dass zur Gewinnung grenzflächenaktiver Rhamnolipide durch Züchtung von Mikroorganismen mehrere Arten der Gattung Pseudomonas in Frage kommen, indem verschiedene Arten, wie Pseudomonas aeruginosa, Pseudomonas fluorescens, Pseudomonas putrida, Pseudomonas cruciviae, Pseudomonas boreopolis, Pseudomonas oleovorans sowie künstlichen oder natürlichen Mutanten dieser Stämme, zur Bildung von Rhamnolipiden befähigt sind; dies gilt grundsätzlich auch für das erfindungsgemässe Verfahren, wobei aber die Stämme der Art Pseudomonas aeruginosa bzw. die natürlichen oder künstlichen Mutanten dieser Art bevorzugt werden.

Für das erfindungsgemässe Verfahren geeignete Mikroorganismen der Art Pseudomonas aeruginosa sind in verschiedenen Stämmen bekannt. Vorzugsweise wird ein neuer Stamm dieser Art verwendet, der als Rsan ver bezeichnet wird und am 31. Mai 1983 bei der DSM, Deutsche Sammlung von Mikroorganismen, Grisebachstr.8, D-3400 Göttingen, mit dem Zeichen DSM 2659 (Rsan ver) hinterlegt wurde. Die wissenschaftliche Beschreibung des Stammes ist im Ergebnisprotokoll der Hinterlegung festgehalten.

Pseudomonas aeruginosa und insbesondere Rsan ver sind bei Züchtung in geeigneten Medien und unter geeigneten Bedingungen zur Bildung von Biotensid befähigt, das die Oberflächenspannung (ST) und die Grenzflächenspannung (IT) der zellfreien Kulturflüssigkeit signifikant senkt, z. B. ST von über 70 $mN \cdot m^{-1}$ auf etwa 30 $mN \cdot m^{-1}$ oder weniger bzw. IT von über 25 $mN \cdot m^{-1}$ auf 1 $mN \cdot m^{-1}$ oder weniger.

Erfindungsgemäss wurde festgestellt, dass das Medium zur wirtschaftlichen Gewinnung von Biotensid mit derartigen Eigenschaften bestimmte Bedingungen erfüllen muss und die Züchtung in kontinuierlicher Kultur zu erfolgen hat, wie im folgenden erläutert.

Da es sich bei den erfindungsgemäss verwendeten Mikroorganismen um Pseudomonas-Arten     handelt, gilt zunächst, dass zur Durchführung des erfindungsgemässen Verfahrens abgesehen von den nachfolgend erläuterten speziellen Bedingungen unter den für diese Mikroben geeigneten allgemeinen Kulturbedingungen (Temperatur, pH-Wert, essentielle Wachstumskomponenten, Spurenelemente) gearbeitet werden kann.

Beispielsweise ist ein Temperaturbereich von 20-40°C für die Züchtung geeignet und ein solcher von 27-34°C, insbesondere von 31-33°C, bevorzugt. Der pH-Wert des Mediums liegt allgemein zwischen 4 und 9, vorzugsweise zwischen 5 und 7,5 und insbesondere im Bereich von 6,0 bis 6,8. Ein pH-Wert von etwa 6,25 ist besonders vorteilhaft.

Die essentiellen Wachstumskomponenten für Pseudomonas-Stämme einschliesslich Rsan ver sind ausser dem bei aerober Kultur grundsätzlich vorhandenen Sauerstoff die Elemente Kohlenstoff, Stickstoff, Schwefel, Phosphor, Natrium, Kalium, Magnesium und Calcium; ferner werden für die Züchtung die Spurenelemente Fe, Zn, Mn, B, Co, Cu und Mo benötigt.

Die genannten Hauptelemente und Spurenstoffe werden wie üblich in assimilierbarer Form und typisch in Mengen von 1-50 g/l C, je 100-2000 mg/l N, S, P, Na und K sowie je 10-500 mg/l Mg und Ca benötigt; die Spurenelemente Zn, Mn, Mo, B, Co und Cu werden meist in Mengen von 0,01 bis 1 mg/l eingesetzt und die Menge an Eisen kann zwischen Spurenanteilen und solchen von 100 mg/l variiert werden. Wie weiter unten erläutert, sind erfindungsgemäss bestimmte Limitierungen erforderlich.

Als Quelle für die Wachstumskomponenten einschliesslich Spurenelemente können die hierfür üblichen Substanzen, z. B. Nitrate, Ammoniumsalze, Sulfate, Phosphate, Metallhalogenide bzw. entsprechende Basen oder Säuren verwendet werden. Die Wahl der Kohlenstoffquelle ist insofern nicht kritisch, als erfindungsgemäss nicht nur Kohlenwasserstoffe, sondern auch Kohlenhydrate geeignet sind und letztere häufig vorteilhafter sind; vorzugsweise wird mit homogenen wässrigen Medien und dementsprechend wasserlöslichen Kohlenhydraten, wie Zuckern, als Kohlenstoffquelle gearbeitet. Glucose ist ein bevorzugtes Beispiel für die Kohlenstoffquelle.

Zur Durchführung des erfindungsgemässen Verfahrens wird unter aeroben Bedingungen in einer kontinuierlichen Submerskultur gearbeitet, d. h. allgemein in einem praktisch konstanten Kulturvolumen, dem fortlaufend frisches Kulturmedium zugeleitet und von dem fortlaufend Kulturmasse, d.h. eine Mischung aus Zellmasse (Biomasse) und einer das verbrauchte Medium und die extrazellularen Metabolite enthaltenden Kulturbrühe, abgezogen wird.

Das Mass (Volumen pro Zeit) der Zuleitung, das bei stetigem Betrieb natürlich gleich dem Mass der Abführung ist, wird als Fraktion des konstanten Volumens pro Zeiteinheit ausgedrückt und als Verdünnungsrate bezeichnet. Eine Verdünnungsrate von beispielsweise $0,1 \cdot h^{-1}$ bedeutet, dass pro Stunde 10 % des konstanten Kulturvolumens durch frisches Medium ersetzt werden, z. B. 1 Liter Medium pro Stunde in einem Reaktor mit einer Arbeitskapazität von 10 Liter.

In diesem Zusammenhang wurde gefunden, dass Verdünnungsraten von über $0,3 \cdot h^{-1}$ zu einer signifikanten Abnahme der Biotensidproduktion führen und dass die höchste Biotensidproduktivität mit dem erfindungsgemässen Verfahren häufig bei Verdünnungsraten unter $0,2 \cdot h^{-1}$ erzielt wird. Demgemäss werden erfindungsgemäss Verdünnungsraten von unter $0,3 \cdot h^{-1}$ und insbesondere unter $0,2 \cdot h^{-1}$ bevorzugt.

Da das Zielprodukt des erfindungsgemässen Verfahrens das extrazellulare Biotensid ist, sind die mikrobiellen Zellen ("Biomasse") ein Nebenprodukt; im allgemeinen ist es daher vorteilhaft, wenn der Biotensidanteil der Kulturmasse möglichst gross und der Biomasseanteil möglichst gering gehalten wird.

- 7 -

0135099

In Richtung auf dieses Ziel wurde gefunden, dass sich das Verhältnis von Biotensidanteil zu Biomasseanteil dadurch erhöhen lässt, dass das Wachstum der Mikroorganismen mindestens zweifach begrenzt wird, d. h. dass das Kulturmedium zwei oder mehr essentielle Wachstumsstoffe nur in limitierenden Anteilen enthält.

Der Anteil eines Wachstumsstoffes am Kulturmedium wird dann als limitierend bezeichnet, wenn eine Verminderung des Anteiles des Wachstumsstoffes am Kulturmedium zu einer merklichen Abnahme der Biomasseproduktion führt.

Beim Arbeiten in kontinuierlicher Kultur kann dies zweckmässig dadurch festgestellt werden, dass das verbrauchte und abgezogene Medium den limitierenden Wachstumsstoff bzw. die limitierenden Wachstumsstoffe nur noch in signifikant verringerten Anteilen enthält. Im allgemeinen gilt hier ein Anteil dann als "signifikant verringert", wenn der Gehalt des verbrauchten Mediums an limitierendem Wachstumsstoff auf weniger als die Hälfte und in der Regel auf weniger als 1 % des entsprechenden Anteiles des frischen Mediums abgesunken ist. Im bevorzugten Fall ist ein limitierender Wachstumsstoff im verbrauchten Medium mit den normalen Betriebsüberwachungsmethoden nicht mehr nachweisbar.

Hieraus ergibt sich zunächst, dass der Grad der Limitierung in gewissen Grenzen variiert werden kann. Im allgemeinen ist eine ausgeprägte Limitierung, d. h. eine solche, die eine praktisch vollständige, d. h. mindestens 99 %ige und vorzugsweise unter die Nachweisbarkeitsgrenze führende Verminderung des Gehaltes an limitierendem Wachstumsstoff im verbrauchten Medium ergibt, erfindungsgemäss bevorzugt.

Vorzugsweise ist das frische Kulturmedium bezüglich Stickstoff und Eisen oder/und Magnesium limitiert. Eine Limitierung durch Phosphor ist weniger vorteilhaft; hier kann sogar ein gewisser Ueberschuss zweckmässig sein. Allgemein wird ein C/P-Verhältnis von weniger als 16 bevorzugt. Die Limitierung durch C liegt im Rahmen der Erfindung. Eine Limitierung durch Na, K, Cl und andere Spurenelemente als Eisen wird nicht bevorzugt. Vorzugsweise liegt der Wert des C/N-Verhältnisses zwischen 8 und 30, insbesondere zwischen 11 und 22, vorzugsweise bei etwa 18. Dies bedeutet, mit anderen Worten, dass die C-Menge im frischen Medium vorzugsweise 8-30mal, bzw. 11-22mal und z.B. 18mal grösser ist, als die N-Menge, und zwar jeweils auf Gewichtsbasis; Nitrate werden dabei als N-Quelle häufig gegenüber Ammoniumsalzen bevorzugt.

Ferner liegt der Wert des C/Fe-Verhältnisses beim erfindungsgemässen Verfahren meist über 1500, vorzugsweise über 3000 und typisch zwischen 3000 und 80'000. Besonders gute Ergebnisse werden z. B. mit 27,5 µg $FeSO_4 \cdot 7H_2O$ pro Gramm Glucose (C-Quelle) erhalten. Da diese bevorzugten Fe-Anteile verhältnismässig sehr gering sind, ist die Verwendung komplexer Zusätze, die wie Hefeextrakt und andere mehr oder weniger natürliche Gemische unterschiedliche und dementsprechend schlecht kontrollierbare Anteile an essentiellen Wachstumsstoffen enthalten, weniger zweckmässig. Allgemein werden für die Erfindung Minimalmedien, d. h. synthetische Kulturmedien mit relativ genau definierter Zusammensetzung, bevorzugt.

Die beim erfindungsgemässen Verfahren aus der kontinuierlichen Kultur fortlaufend abgeführte oder abgezogene Kulturmasse ist eine Mischung aus verbrauchtem Medium und Zell- oder Biomasse; diese Mischung enthält das metabolitisch gebildete Biotensid, das normalerweise ein Rhamnosid bzw. ein Rhamnolipid ist. Der Lipidteil des erfindungsgemäss als Biotensid gebildeten Glycolipids wird nicht als kritisch angesehen.

Das Rhamnosid, das beim erfindungsgemässen Verfahren typisch in Anteilen von 0,5 bis 2 g/l erhalten werden kann, ist normalerweise in der Kulturmasse, und zwar überwiegend in der Kulturbrühe bzw. im verbrauchten Kulturmedium gelöst. Dementsprechend wird die Rhamnosidlösung von der Kulturmasse getrennt, z. B. durch Zentrifugieren oder Filtrieren, und kann in dieser Form einer bestimmungsgemässen Tensidverwendung zugeführt werden, z. B. zur verstärkten Erdölgewinnung.

Zur Verwendung für andere Zwecke, z. B. als Suspensions-, Dispergier-, Emulgier- oder Netzmittel für typische technische Verfahren oder/und kommerzielle Produkte kann das Biotensid durch Fällung oder/und Extraktion aus der von der Biomasse abgetrennten Kulturbrühe isoliert werden. Zur Fällung können Mittel, wie Zinkchlorid oder Calciumchlorid, eingesetzt werden. Für die Extraktion sind organische Lösungsmittel, wie Diethyläther, Chloroform und Ethylacetat, verwendbar. Ferner kann zunächst durch Fällung ein Rohprodukt gewonnen und dieses - gegebenenfalls als Suspension in einem Puffermedium - mit organischem Lösungsmittel extrahiert werden. Aus dem Extrakt kann durch Verdampfen des Lösungsmittels praktisch reines Biotensid gewonnen werden; das Extraktionsergebnis kann in gewissen Grenzen durch den pH-Wert beeinflusst werden.

Aus der Biomasse kann, gegebenenfalls nach einer Behandlung zur Zerstörung der Zellwände, weiteres Biotensid bzw. zusätzliche Biotensidlösung gewonnen und ähnlich wie oben beschrieben verwendet bzw. aufgearbeitet werden.

Die Zeichnungen dienen der weiteren Erläuterung der Erfindung anhand eines Ausführungsbeispiels. Es zeigen:

Fig. 1  das Schema der Durchführung einer Ausführungsform des Verfahrens und

Fig. 2  das Profil von Produktivität/Verdünnungsrate bei der Durchführung des Verfahrens.

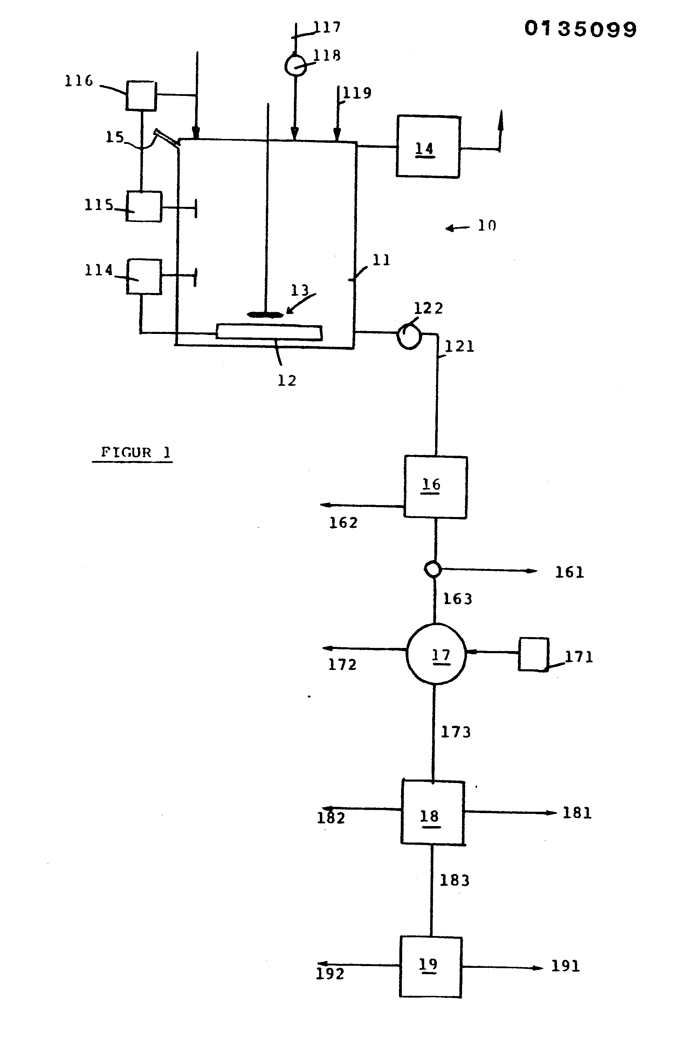

Zur Durchführung des in Fig. 1 schematisch dargestellten Verfahrens wird für die Kultivierungsstufe 10 ein Bioreaktor 11 verwendet, der mit einer Heizung 12 und einem Turbinenrührer 13 ausgerüstet und mit einem Schaumabscheider 14 versehen ist. Die Schleuse 15 kann zum Animpfen einer im Reaktor 11 vorgelegten Menge Kulturmedium beim Anlaufen des Reaktors und zur Entnahme von Proben verwendet werden.

Mit dem Temperaturregler 114 wird die Heizung 12 gesteuert, um die Temperatur des Reaktorinhaltes auf einem praktisch konstanten Wert zu halten. Mit dem Messgerät 115 wird der pH-Wert laufend überwacht und gegebenenfalls mit Hilfe einer gesteuerten Säure/Lauge-Zugabeeinrichtung 116 auf dem Sollwert gehalten. Frisches Kulturmedium wird durch die Zuleitung 117 in den Reaktor 11 eingespeist. Die Zuflussrate der Einspeisung des Mediums kann mit dem Zuflussventil 118 gesteuert werden. Ferner wird durch die Leitung 119 Sauerstoff, Luft oder ein anderes sauerstoffhaltiges Gasgemisch in einer zur Erhaltung aerober Bedingungen ausreichenden Menge eingespeist.

Bei kontinuierlichem Betrieb wird Kulturmasse über die Leitung 121 abgezogen; mit dem Ventil 122 kann die Abflussrate der Kulturmasse eingestellt bzw. die Leitung 121 beim Anlaufen des Reaktors gesperrt werden.

Ferner sind (nicht dargestellte) Einrichtungen zum Sterilisieren des Reaktors und zur Messung weiterer Parameter, wie Sauerstoffgehalt, vorgesehen.

<u>Beispiel</u>

Zur Einleitung des Verfahrens wurde ein vorgängig sterilisierter 5-Liter-Reaktor 11 für kontinuierlichen Betrieb mit frischem sterilem Kulturmedium gefüllt, das die in der folgenden Tabelle I angegebene Zusammensetzung hatte.

<u>TABELLE I</u>

| <u>Komponente</u> | <u>Konzentration (Liter$^{-1}$)</u> |
|---|---|
| Glucose | 18,2 g |
| $NaNO_3$ | 2,5 g |
| $MgSO_4 \cdot 7H_2O$ | 0,2 g |
| KCl | 1,0 g |
| NaCl | 1,0 g |
| $H_3PO_4$ (85 %ig) | 1,0 ml |
| $CaCl_2 \cdot 2H_2O$ | 50 mg |
| $FeSO_4 \cdot 7H_2O$ | 0,5 mg |
| $ZnSO_4 \cdot 7H_2O$ | 1,5 mg |
| $MnSO_4 \cdot 2H_2O$ | 1,5 mg |
| $H_3BO_3$ | 0,3 mg |
| $CuSO_4 \cdot 5H_2O$ | 0,15 mg |
| $CoCl_2 \cdot 6H_2O$ | 0,15 mg |
| $Na_2MoO_4 \cdot 2H_2O$ | 0,10 mg |
| entmineralisiertes Wasser | Rest |

Nach Einschalten des Rührers (1500 U/min) wurde die Temperatur des Mediums auf 37°C gebracht und mit der Temperatursteuerung 114 auf diesem Wert gehalten. Der pH-Wert wurde mit wässriger normaler KOH-Lösung auf 6,5 eingestellt und von der Messeinrichtung 115 überwacht. Das Arbeitsvolumen betrug 1,5 Liter. Nach dem Beimpfen des Kulturmediums mit 250-300 ml Inoculum von Rsan ver (DSM 2659) wurde der Reaktorinhalt 8 Std. mit geschlossenem Abzugsventil 122 bei der angegebenen Temperatur gehalten und unter fortlaufender Frischluftzuführung (2,25 Liter/min) durch die Belüftungsleitung und Luftableitung durch den Schaumabscheider 14 (Betrieb mit 2000 U/min) aerob gehalten.

Nach Beendigung der Anlaufzeit wurde unter sonst gleichen Bedingungen das Ventil 122 zum Abziehen von 150 ml Kulturmasse
pro Stunde durch die Abführleitung 121 geöffnet. Gleichzeitig
wurde das Ventil 118 in der Zuführleitung 117 soweit geöffnet, dass pro Stunde 150 ml frisches Kulturmedium in den Reaktor 11 strömten.

In der ersten Trennstufe 16, z. B. einer Zentrifuge oder einem
Filter, wurde die Kulturmasse in Biomasse und zellfreie Kulturbrühe getrennt. Die Biomasse wird über 162 abgeführt und gegebenenfalls nachbehandelt bzw. verwertet.

Die zellfreie Kulturbrühe enthält das Biotensid gelöst im verbrauchten Kulturmedium und kann entweder über 161 zur direkten
Verwendung in dieser Lösungsform abgeführt oder über 163 in
eine zweite Trennstufe 17 geführt und dort durch Fällung unter
Zugabe von Fällungsmittel aus 171 oder durch Extraktion unter
Zugabe von Lösungsmittel aus 171 aus der Kulturbrühe gewonnen
werden, deren von Biotensid befreiter Rest über 172 abgeführt
und gewünschtenfalls zur Regeneration bzw. Herstellung von
frischem Kulturmedium rezirkuliert werden kann.

Das in der Trennstufe 17 gewonnene Präzipitat bzw. der dort
gewonnene Extrakt kann über 173 in eine erste Reinigungsstufe
18 überführt und dort je nach Art des in Stufe 17 gewonnenen
Rohmaterials resuspendiert und extrahiert oder vom Lösungsmittel befreit werden. Dementsprechend kann in der Reinigungsstufe 18 entweder ein gereinigtes Biotensidprodukt erhalten und
über 181 gewonnen oder über 183 in eine zweite Reinigungsstufe
19 überführt und dort über 191 gewonnen werden. Ueber 182 bzw.
192 werden die jeweiligen Rückstände der entsprechenden Reinigungsstufen abgeführt.

Die fortlaufende Messung der Zusammensetzung des durch die Leitung 121 zusammen mit Biomasse abgeführten verbrauchten Nährmediums zeigt, dass das frische Medium bezüglich C, N und Fe limitiert ist, indem die Anteile dieser Wachstumsstoffe am verbrauchten Medium praktisch nicht mehr nachweisbar waren.

Das Biotensid wurde in einer Ausbeute entsprechend etwa 80 mg Rhamnose-Anteil pro Gramm Substratkohlenstoff erhalten, was beim verwendeten Medium einem Rhamnosid-Anteil von 640 mg/Liter bzw. einem Biotensid-Anteil von 1-1,5 g/Liter entspricht.

Die Oberflächenspannung (ST) der durch die Leitung 121 abströmenden Biotensidlösung lag unter 30 $(mN \cdot m^{-1})$, die Grenzflächenspannung (IT) unter 1 $(mN \cdot m^{-1})$, bestimmt bei Raumtemperatur (20°C) nach der üblichen Ringmethode und mit einem modifizierten Dunuoy-Tensiometer, wie es unter der Bezeichnung "Auto-Tensiomat" von der Firma Fisher, USA, erhältlich ist.

Das gewonnene Biotensid ist biologisch leicht abbaubar und kann insbesondere dort mit Vorteil verwendet werden, wo physiologisch unbedenkliche Tenside benötigt werden, z. B. in der Lebensmittelverarbeitung bzw. der Herstellung von physiologisch unbedenklichen Produkten.

Die hervorragende Wirksamkeit von erfindungsgemäss erhältlichen Biotensidlösungen für verstärkte Erdölgewinnung ist beispielsweise daran zu erkennen, dass die Biotensidlösung bei einer Konzentration von 0,3 g/100 ml einen mindestens gleichwertigen Oel-Spüleffekt (33 % Restölgewinnung) zeigt, wie ein kommerziell für diese Verwendung erhältliches synthetisches Tensid (PYRONATE 40, ein von der Firma Witco Co., USA, erhältliches Natriumpetrolsulfonat) in einer zehnmal höheren Konzentration (3 g/100 ml; 31 % Restölgewinnung), und zwar bei Bewertung nach dem sogenannten Sandpack-Flooding-Test.

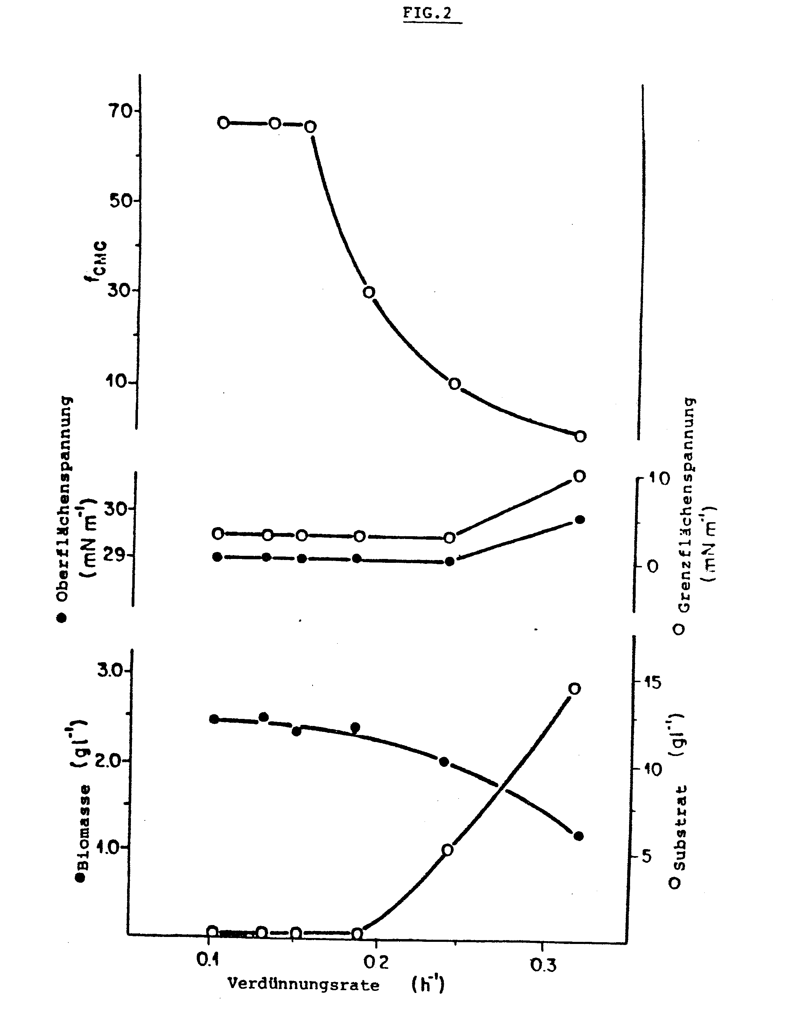

Bei dem in Fig. 2 dargestellten Produktivitäts-Verdünnungsraten-Profil ist auf der gemeinsamen Abszisse die in verschiedenen Betriebsläufen verwendete Verdünnungsrate mit der Dimension $[h^{-1}]$ aufgetragen. Die linke Ordinate zeigt im untersten Teil die erhaltene Biomasse (in Gramm pro Liter), im mittleren Teil die Oberflächenspannung der Kulturbrühe (in Millinewton pro Meter) und im obersten Teil den von der kritischen Micelkonzentration (CMC) abgeleiteten Wert f, der zur Bewertung der Biotensidkonzentration dient.

Die rechte Ordinate zeigt im unteren Teil die Substratkonzentration (in Gramm pro Liter) und im mittleren Teil die Grenzflächenspannung (in Millinewton pro Meter).

Aus Fig. 2 ist zu erkennen, dass bei Verdünnungsraten von unter 0,3 und insbesondere unter 0,2 bessere Ergebnisse bezüglich Tensideigenschaften und Tensidausbeute erzielt werden können.

Patentansprüche

1. Verfahren zur Herstellung von als Tenside verwendbaren Rhamnolipiden durch Kultivieren von Mikroorganismen der Gattung Pseudomonas in einem für das Wachstum der Mikroorganismen geeigneten wässrigen Kulturmedium unter Wachstumsbedingungen der Mikroorganismen, dadurch gekennzeichnet, dass

(a) die Mikroorganismen in einer kontinuierlichen Submerskultur unter aeroben Bedingungen und mit fortlaufender Zuführung von frischem Kulturmedium sowie fortlaufender Abführung einer Kulturmasse aus teilweise verbrauchtem Kulturmedium und gebildeten Tensiden, sowie gegebenenfalls mikrobiellen Zellen gezüchtet werden, und

(b) das Kulturmedium eine zur Wachstumsbegrenzung durch mindestens zweifache Limitierung essentieller Wachstumsstoffe geeignete Zusammensetzung aufweist und

(c) Gegebenenfalls von der Kulturmasse eine Lösung der als Metabolit der Mikroorganismen gebildeten rhamnolipide abgetrennt wird.

2. Verfahren nach Anspruch 1, dadurch gekennzeichnet, dass Pseudomonas-Mikroorganismen der Art Pseudomonas aeruginosa verwendet werden.

3. Verfahren nach Anspruch 2, dadurch gekennzeichnet, dass Pseudomonas aeruginosa vom Stamm DSM 2659 (Rsan ver) verwendet werden.

4. Verfahren nach einem der Ansprüche 1-3, dadurch gekennzeichnet, dass das Kulturmedium bezüglich Stickstoff limitiert ist.

5. Verfahren nach einem der Ansprüche 1-4, dadurch gekennzeichnet, dass das Kulturmedium bezüglich Eisen limitiert ist.

6. Verfahren nach einem der Ansprüche 1-5, dadurch gekennzeichnet, dass das Kulturmedium bezüglich Magnesium limitiert ist.

7. Verfahren nach einem der Ansprüche 1-6, dadurch gekennzeichnet, dass in kontinuierlicher Kultur mit einer Verdünnungsrate von unter $0,3 \, h^{-1}$ gearbeitet wird.

8. Verfahren nach Anspruch 7, dadurch gekennzeichnet, dass mit einer Verdünnungsrate von unter $0,2 \, h^{-1}$ gearbeitet wird.

9. Verfahren nach einem der Ansprüche 1-8, dadurch gekennzeichnet, dass die in Schritt (c) erhaltene Lösung zur Isolierung des Rhamnolipids durch Fällung oder/und Affinitätschromatographie aufgearbeitet wird.

10. Verfahren nach einem der Ansprüche 1-9, dadurch gekennzeichnet, dass ein homogenes wässriges Kulturmedium verwendet wird, das praktisch frei ist von anteilsmässig unbestimmten Wachstumsstoffen.

11. Verfahren nach einem der Ansprüche 1-10, dadurch gekennzeichnet, dass die Kohlenstoffquelle für das Wachstum der Mikroorganismen im Kulturmedium mindestens teilweise aus wasserlöslichem Kohlenhydrat, z. B. Glucose, besteht.

12. Verfahren nach einem der Ansprüche 1-11, dadurch gekennzeichnet, dass das Kulturmedium bezüglich Phosphor nicht limitiert ist.

13. Verfahren nach einem der Ansprüche 1-12, dadurch gekennzeichnet, dass das Kohlenstoff/Phosphor-Verhältnis im Kulturmedium kleiner als 16 ist.

0135099

14. Verfahren nach einem der Ansprüche 1-13, dadurch gekennzeichnet, dass das Kohlenstoff/Stickstoff-Verhältnis im
Kulturmedium zwischen 8 und 30 liegt.

15. Verfahren nach Anspruch 14, dadurch gekennzeichnet, dass
das Kohlenstoff/Stickstoff-Verhältnis im Kulturmedium etwa
18 beträgt.

0135099

FIGUR 1

0135099

FIG.2